# EUROPEAN PATENT SPECIFICATION

(11) **EP 1 315 517 B1**
(45) Date of publication and mention of the grant of the patent: **20.10.2010**
(21) Application number: 01965715.4
(22) Date of filing: 04.09.2001
(51) Int. Cl.: A61K 39/00, C07K 14/775

(54) **MIMETIC PEPTIDES FOR EPITOPE OF APOLIPOPROTEIN B-100, CONCATEMER AND MODIFIED PEPTIDES THEREOF, AND THE VACCINE COMPOSITION COMPRISING THE SAME**
MIMETISCHE PEPTIDEN FÜR EPITOP VON APOLIPOPROTEIN B-100, CONCATEMER UND MODIFIZIERTE PEPTIDE DAVON, UND DIE IMPFSTOFFZUSAMMENSETZUNG MIT SOLCHEN PEPTIDEN
PEPTIDES MIMETIQUES POUR L'EPITOPE DE L'APOLIPOPROTEINE B-100, CONCATEMERES ET SES PEPTIDES MODIFIES, ET COMPOSITION DE VACCIN LES COMPRENANT

(30) Priority: 04.09.2000 KR 2000052055; 04.09.2001 KR 2001054005
(43) Date of publication of application: 04.06.2003
(73) Proprietor: Kim, Hyo-Joon, 425-040 Ansan (KR)
(72) Inventor: KIM, Hyo-Joon, 425-040 Ansan (KR); JOUNG, Hae-Jung, 134-024 Seoul (KR)
(74) Representative: Long, Giorgio
(86) International application number: PCT/KR2001/001492
(87) International publication number: WO 2002/020040

(56) References cited:
- WO-A-01/64008
- WO-A-99/45020
- CORSINI A ET AL: "Receptor binding activity of lipid recombinants of apolipoprotein B-100 thrombolytic fragments." JOURNAL OF LIPID RESEARCH. DEC 1987, vol. 28, no. 12, December 1987 (1987-12), pages 1410-1423, XP002310083 ISSN: 0022-2275
- TAKI T ET AL: "PREPARATION OF PEPTIDES WHICH MIMIC GLYCOPHINGOLIPIDS BY USING PHAGE PEPTIDE LIBRARY AND THEIR MODULATION ON BETA-GALACTOSIDASE ACTIVITY" FEBS LETTERS, ELSEVIER SCIENCE PUBLISHERS, AMSTERDAM, NL, vol. 418, no. 1/2, 1997, pages 219-223, XP000941485 ISSN: 0014-5793
- COUET J ET AL: "IDENTIFICATION OF PEPTIDE AND PROTEIN LIGANDS FOR THE CAVEOLIN-SCAFFOLDING DOMAIN. IMPLICATIONS FOR THE INTERACTION OF CAVEOLIN WITH CAVEOLAE-ASSOCIATED PROTEINS" JOURNAL OF BIOLOGICAL CHEMISTRY, AMERICAN SOCIETY OF BIOLOGICAL CHEMISTS, BALTIMORE, MD, US, vol. 272, no. 10, 7 March 1997 (1997-03-07), pages 6525-6533, XP000941496 ISSN: 0021-9258
- MILNE R W ET AL: "Monoclonal antibodies against human low density lipoprotein. Stoichiometric binding studies using Fab fragments." FEBS LETTERS. 6 SEP 1982, vol. 146, no. 1, 6 September 1982 (1982-09-06), pages 97-100, XP002310084 ISSN: 0014-5793
- TIKKANEN M ET AL: "The recognition domain for the low density lipoprotein cellular receptor is expressed once on each lipoprotein particle." BIOCHEMICAL AND BIOPHYSICAL RESEARCH COMMUNICATIONS. 31 JAN 1985, vol. 126, no. 2, 31 January 1985 (1985-01-31), pages 773-777, XP009041525 ISSN: 0006-291X
- HOSPATTANKAR A V ET AL: "Identification of low density lipoprotein receptor binding domains of human apolipoprotein B-100: a proposed consensus LDL receptor binding sequence of apoB-100." BIOCHEMICAL AND BIOPHYSICAL RESEARCH COMMUNICATIONS. 30 SEP 1986, vol. 139, no. 3, 30 September 1986 (1986-09-30), pages 1078-1085, XP009041518 ISSN: 0006-291X
- MARCEL Y L ET AL: "Mapping of human apolipoprotein B antigenic determinants." ARTERIOSCLEROSIS (DALLAS, TEX.) 1987 MAR-APR, vol. 7, no. 2, March 1987 (1987-03), pages 166-175, XP009041524 ISSN: 0276-5047
- WATTS G F ET AL: "Genotypic associations of the hepatic secretion of VLDL apolipoprotein B-100 in obesity." JOURNAL OF LIPID RESEARCH. MAR 2000, vol. 41, no. 3, March 2000 (2000-03), pages 481-488, XP002310088 ISSN: 0022-2275
- AZAIN M J ET AL: "Contributions of fatty acid and sterol synthesis to triglyceride and cholesterol secretion by the perfused rat liver in genetic hyperlipemia and obesity." THE JOURNAL OF BIOLOGICAL CHEMISTRY. 10 JAN 1985, vol. 260, no. 1, 10 January 1985 (1985-01-10), pages 174-181, XP002310089 ISSN: 0021-9258
- BAILEY J M: "Cholesterol vaccines." SCIENCE. 20 MAY 1994, vol. 264, no. 5162, 20 May 1994 (1994-05-20), pages 1067-1068, XP001204252 ISSN: 0036-8075
- NILSSON J ET AL: "Immunization with homologous oxidized low density lipoprotein reduces neointimal formation after balloon injury in hypercholesterolemic rabbits" JOURNAL OF THE AMERICAN COLLEGE OF CARDIOLOGY, XX, XX, vol. 30, no. 7, December 1997 (1997-12), pages 1886-1891, XP002287789 ISSN: 0735-1097
- PALINSKI W ET AL: "IMMUNOZATION OF LOW DENSITY LIPOPROTEIN (LDL) RECEPTOR-DEFICIENT RABBITS WITH HOMOLOGOUS MALONDIALDEHYDE-MODIFIED LDL REDUCES ATHEROGENESIS" PROCEEDINGS OF THE NATIONAL ACADEMY OF SCIENCES OF USA, NATIONAL ACADEMY OF SCIENCE. WASHINGTON, US, vol. 92, January 1995 (1995-01), pages 821-825, XP002941307 ISSN: 0027-8424
- GAW A ET AL: "Apolipoprotein B metabolism in primary and secondary hyperlipidaemias." CURRENT OPINION IN LIPIDOLOGY. JUN 1996, vol. 7, no. 3, June 1996 (1996-06), pages 149-154, XP009041220 ISSN: 0957-9672
- PACKARD C J: "Understanding coronary heart disease as a consequence of defective regulation of apolipoprotein B metabolism." CURRENT OPINION IN LIPIDOLOGY. JUN 1999, vol. 10, no. 3, June 1999 (1999-06), pages 237-244, XP009041229 ISSN: 0957-9672
- ALVING C. AND WASSEF N.: 'Naturally occurring antibodies to cholesterol: a new theory of LDL cholesterol metabolism' IMMUNOLOGY TODAY vol. 20, no. 8, 1999, pages 362 - 366, XP004177364
- ALVING C. ET AL: 'Immunization with cholesterol-rich liposomes induces anti-cholesterol antibodies and reduces diet-induced hypercholesterolemia and plaque formation' J. LAB. CLIN. MED. vol. 127, no. 1, 1996, pages 40 - 49, XP001095396
- ALVING C. ET AL: 'Antibodies to cholesterol: biological implications of antibodies to lipids' CURR. TOP. MICROBIOL. IMMUNOL. vol. 210, 1996, pages 181 - 186, XP002923267
- SOUED M. AND MANSBACH C.: 'Chylomicron remnant uptake by enterocytes is receptor dependent' AM. J. PHYSIOL. vol. 270, 1996, pages G203 - G212, XP002980789
- YOUNG S.: 'Recent progress in understanding apolipoprotein B' CIRCULATION vol. 82, no. 5, 1990, pages 1574 - 1594, XP002980787
- KNOTT T. ET AL: 'Complete protein sequence and identification of structural domains of human apolipoprotein B' NATURE vol. 323, no. 6090, 1986, pages 734 - 738, XP002133890
- STADLER M. ET AL: 'Mimotope and anti-idiotype vaccines to induce an anti-IgE response' INT. ARCH. ALLERGY IMMUNOL. vol. 118, no. 2-4, 1999, pages 119 - 121, XP000916451
- STEWARD M. ET AL: 'A mimotope from a solid-phase peptide library induces a measles virus-neutralization and protective antibody response' J. VIROL. vol. 69, no. 12, 1995, pages 7668 - 7673, XP002980775

### Sequence Listing

<110> KIM, Hyo Joon
<120> Mimetic peptides for epitope of apolipoprotein B-100, concatemer and modified peptides thereof, and the vaccine composition comprising the same
<130> SJPA0103
<150> KR10-2000-0052055
   <151> 2000-09-04
<160> 16
<170> KopatentIn 1.71
<210> 1
   <211> 15
   <212> PRT
   <213> Artificial Sequence
<220>
   <223> mimetic peptide for apolipoprotein B-100
<400> 1
   Arg Asn Val Pro Pro Ile Phe Asn Asp Val Tyr Trp Ile Ala Phe
   1               5                   10                  15
<210> 2
   <211> 15
   <212> PRT
   <213> Artificial Sequence
<220>
   <223> mimetic peptide for apolipoprotein B-100
<400> 2
   Arg Phe Arg Gly Leu Ile Ser Leu Ser Gln Val Tyr Leu Asp Pro
   1 5 10 15
<210> 3
   <211> 15
   <212> PRT
   <213> Artificial Sequence
<220>
   <223> mimetic peptide for apolipoprotein B-100
<400> 3
   Ser Val Cys Gly Cys Pro Val Gly His His Asp Val Val Gly Leu
   1 5 10 15
<210> 4
   <211> 23
   <212> DNA
   <213> Artificial Sequence
<220>
   <223> oligonucleotide for the construction of BL or LB cassette
<400> 4
   tcgaccgtaa tgttcctcct atc 23
<210> 5
   <211> 28
   <212> DNA
   <213> Artificial Sequence
<220>
   <223> oligonucleotide for the construction of BL or LB cassette
<400> 5
   atcattgaag ataggaggaa cattacgg 28
<210> 6
   <211> 29
   <212> DNA .
   <213> Artificial Sequence
<220>
   <223> oligonuclotide for the construction of LB cassette
<400> 6
   ttcaatgatg tttattggat tgcattcta 29
<210> 7
   <211> 24
   <212> DNA
   <213> Artificial Sequence
<220>
   <223> oligonucleotide for the construction of LB cassette
<400> 7
   agcttagaat gcaatccaat aaac 24
<210> 8
   <211> 28
   <212> DNA
   <213> Artificial Sequence
<220>
   <223> oligonucleotide for the construction of BL cassette
<400> 8
   ttcaatgatg tttattggat tgcattcc 28
<210> 9
   <211> 23
   <212> DNA
   <213> Artificial Sequence
<220>
   <223> oligonucleotide for the construction of BL cassette
<400> 9
   tcgaggaatg caatccaata aac 23
<210> 10
   <211> 24
   <212> DNA
   <213> Artificial Sequence
<220>
   <223> upper strand of the leader cassette
<400> 10
   gatccgatga tgatgacaag atcg 24
<210> 11
   <211> 24
   <212> DNA
   <213> Artificial Sequence
<220>
   <223> lower strand of the leader cassette
<400> 11
   tcgacgatct tgtcatcatc atcg 24
<210> 12
   <211> 6
   <212> PRT
   <213> Artificial Sequence
<220>
   <223> enterokinase cleavage site
<400> 12
   Asp Asp Asp Asp Lys Ile
   1 5
<210> 13
   <211> 52
   <212> DNA
   <213> Artificial Sequence
<220>
   <223> upper strand of the LB cassette
<400> 13
   tcgaccgtaa tgttcctcct atcttcaatg atgtttattg gattgcattc ta 52
<210> 14
   <211> 52
   <212> DNA
   <213> Artificial Sequence
<220>
   <223> lower strand of the LB cassette
<400> 14
   agcttagaat gcaatccaat aaacatcatt gaagatagga ggaacattac gg 52
<210> 15
   <211> 51
   <212> DNA
   <213> Artificial Sequence
<220>
   <223> upper strand of the BL cassette
<400> 15
   tcgaccgtaa tgttcctcct atcttcaatg atgtttattg gattgcattc c 51
<210> 16
   <211> 51
   <212> DNA
   <213> Artificial Sequence
<220>
   <223> lower strand of the BL cassette
<400> 16
   tcgaggaatg caatccaata aacatcattg aagataggag gaacattacg g 51

<110> KIM, Hyo Joon
<120> Mimetic peptides for epitope of apolipoprotein B-100, concatemer and derivatives thereof, and the vaccine composition comprising the same
<130> SJPA0103PCT
<150> KR10-2000-0052055
   <151> 2000-09-04
<160> 16
<170> KopatentIn 1.71
<210> 1
   <211> 15
   <212> PRT
   <213> Artificial Sequence
<220>
   <223> mimetic peptide for apolipoprotein B-100
<400> 1
   Arg Asn Val Pro Pro Ile Phe Asn Asp Val Tyr Trp Ile Ala Phe
   1 5 10 15
<210> 2
   <211> 15
   <212> PRT
   <213> Artificial Sequence
<220>
   <223> mimetic peptide for apolipoprotein B-100
<400> 2
   Arg Phe Arg Gly Leu Ile Ser Leu Ser Gln Val Tyr Leu Asp Pro
   1 5 10 15
<210> 3
   <211> 15
   <212> PRT
   <213> Artificial Sequence
<220>
   <223> mimetic peptide for apolipoprotein B-100
<400> 3
   Ser Val Cys Gly Cys Pro Val Gly His His Asp Val Val Gly Leu
   1 5 10 15
<210> 4
   <211> 23
   <212> DNA
   <213> Artificial Sequence
<220>
   <223> oligonucleotide for the construction of BL or LB cassette
<400> 4
   tcgaccgtaa tgttcctcct atc 23
<210> 5
   <211> 28
   <212> DNA
   <213> Artificial Sequence
<220>
   <223> oligonucleotide for the construction of BL or LB cassette
<400> 5
   atcattgaag ataggaggaa cattacgg 28
<210> 6
   <211> 29
   <212> DNA
   <213> Artificial Sequence
<220>
   <223> oligonuclotide for the construction of LB cassette
<400> 6
   ttcaatgatg tttattggat tgcattcta 29
<210> 7
   <211> 24
   <212> DNA
   <213> Artificial Sequence
<220>
   <223> oligonucleotide for the construction of LB cassette
<400> 7
   agcttagaat gcaatccaat aaac 24
<210> 8
   <211> 28
   <212> DNA
   <213> Artificial Sequence
<220>
   <223> oligonucleotide for the construction of BL cassette
<400> 8
   ttcaatgatg tttattggat tgcattcc 28
<210> 9
   <211> 23
   <212> DNA
   <213> Artificial Sequence
<220>
   <223> oligonucleotide for the construction of BL cassette
<400> 9
   tcgaggaatg caatccaata aac 23
<210> 10
   <211> 24
   <212> DNA
   <213> Artificial Sequence
<220>
   <223> upper strand of the leader cassette
<400> 10
   gatccgatga tgatgacaag atcg 24
<210> 11
   <211> 24
   <212> DNA
   <213> Artificial Sequence
<220>
   <223> lower strand of the leader cassette
<400> 11
   tcgacgatct tgtcatcatc atcg 24
<210> 12
   <211> 6
   <212> PRT
   <213> Artificial Sequence
<220>
   <223> enterokinase cleavage site
<400> 12
   Asp Asp Asp Asp Lys Ile
   1 5
<210> 13
   <211> 52
   <212> DNA
   <213> Artificial Sequence
<220>
   <223> upper strand of the LB cassette
<400> 13
   tcgaccgtaa tgttcctcct atcttcaatg atgtttattg gattgcattc ta 52
<210> 14
   <211> 52
   <212> DNA
   <213> Artificial Sequence
<220>
   <223> lower strand of the LB cassette
<400> 14
   agcttagaat gcaatccaat aaacatcatt gaagatagga ggaacattac gg 52
<210> 15
   <211> 51
   <212> DNA
   <213> Artificial Sequence
<220>
   <223> upper strand of the BL cassette
<400> 15
   tcgaccgtaa tgttcctcct atcttcaatg atgtttattg gattgcattc c 51
<210> 16
   <211> 51
   <212> DNA
   <213> Artificial Sequence
<220>
   <223> lower strand of the BL cassette
<400> 16
   tcgaggaatg caatccaata aacatcattg aagataggag gaacattacg g 51

## Description

### Technical Field

The present invention relates to a vaccine composition for treatment of obesity. More particularly, the present invention is directed to a vaccine composition which comprises mimetic peptide epitope of apolipoprotein B-100, concatemers or modified peptides thereof.

### Background Art

Blood serum lipid is composed of cholesterol, triglycerides(TG), free fatty acid, phospholipid and the like, and exist in blood stream in the form of lipoprotein which is complex of lipid and apolipoprotein.

Of these lipoproteins, low density lipoprotein (LDL) is major carrier for TG and cholesterol. The number of patient suffering from arteriosclerosis, coronary artery disease, or cardiac infarction caused by elevated LDL-cholesterol level in blood, has been considerably increased due to the change of dietary life or other factors.

Therefore, various researches for lowering the level of LDL-cholesterol and for showing the cause of above diseases, have been tried to treat patients suffering from the above diseases.

LDL-cholesterol, a major etiological factor for adult disease related to lipid metabolism, may be converted to the form of high density lipoprotein(HDL) by macrophage. In addition, LDL-cholesterol may also be converted to another material or be converted to the form of bile acid in the liver. (Brown, M.S. and Goldstein, J.L., 1983, Annu. Rev. Biochem., 52:223-261).

Apolipoprotein B-100 is a major protein part of LDL and exists also in very low density lipoprotein (VLDL) and chylomicron. LDL-cholesterol in blood may be removed through phagocytosis by macrophage in case that an antibody in blood stream is induced by recognizing the apolipoprotein B-100, since apolipoprotein B-100 leads LDL particles to bind to LDL-receptors exposed on the cell surface (Dalum I., et al., 1997, Mol. Immunol., 34(16-17): 1113-20).

In case that a macromolecule such as an antibody has been bound to apolipoprotein B-100 which exists on the surface of LDL, lipase such as lipoprotein lipase cannot hydrolize TG and the likes due to the steric hindrance caused by the macromolecule bound to apolipoprotein B-100. Consequently, the formation of free fatty acid, a major factor for obesity, can be inhibited by means of the antibody which can bind to apolipoprotein B-100.

Recently, several researches for lowering LDL-cholesterol level and for inhibiting the outbreak of arteriosclerosis by using a vaccine, have been tried in various animal models such as mouse and rabbit. For example, C.R. Alving reported that cholesterol may be modified by metabolites or its oxidation, and that the modified cholesterol can be a strong antigenic determinants in some cases (Alving, C.R., et al., 1989, Biochem. Soc. Trans., 17(4): 637-9; Alving, C.R., et al., 1996, J. Lab. Clin. Med., 127: 40-49; Alving, C.R., et al., 1996, Curr. Top. Microbiol. Immunol., 210: 181-6).

Furthermore, it has been reported that an endogenous antibody for cholesterol exists in blood serum (Wu, J.T., L.L., 1997, Clin. Lab. Med., 17(3): 595-604, Review). It has also been reported that, in the experiment wherein arteriosclerosis and hypercholesterolemia are induced on rabbit by feeding cholesterol-containing meal, the occurrence of hypercholesterolemia and arteriosclerosis in the rabbit immunized by injecting cholesterol-containing liposome, is suppressed or lowered remarkably than those in control group.

Such antibody induced by cholesterol vaccine, is immunoglobin M (IgM) which binds to VLDL, intermediate density lipoprotein (IDL) and LDL. Based on the above, it is believed that a vaccine for treatment or prevention of hyperlipoidemia or arteriosclerosis caused by high level of cholesterol, will be possible (Bailey, J.M., 1994, Science, 264: 1067-1068; Palinski, W. et al., 1995, Proc. Natl. Acad. Sci. U.S.A., 92(3): 821-5; Wu, R. et al, 1999, Hypertension, 33(1): 53-9).

The present inventors have found that obesity can be effectively prevented by mimetic peptide epitope of apolipoprotein B-100, and based on the above, have developed a vaccine composition for treatment of obesity.

### Disclosure of invention

Therefore, the object of the present invention is to provide an isolated concatemer of peptides as per claim 1 and 2.

Another object of the present invention is to provide a process for preparing the above isolated concatemer of peptides as per claim 6.

Yet another object of the present invention is to provide a vaccine composition for treatment of obesity as per claims 3 to 5.

A peptide library system of phage was employed in order to screen an epitope of human apolipoprotein B-100 bound by monoclonal antibody (MabB23). The screened peptides in the above were mimetic peptides structurally similar to antigenic determinant which can be recognized by the antibody, and these mimetic peptides were synthesized according to the amino acid sequence of the screened peptide.

Peptide library system is a kind of process for searching a three dimensional form of antigenic determinant. That is, DNA fragments which encode random-sequenced peptides are inserted into DNAs which encodes minor coat protein of phage, and then the above DNAs are inserted into RF (Reading frame) DNA and transformed to *E.coli* in order to express them. The peptides expressed on the surface of E.coli, are reacted with antigen in order to screen the peptides structurally similar to the antigenic determinant.

In order to prepare anti-serum, mice was immunized by introducing the above mimetic peptides. It was confirmed that the anti-serum thus obtained recognizes the original apolipoprotein B-100, mimetic peptides and LDL at the same times (*Identification of Antigenic Determinants for the Murine Monoclonal Antibodies Against Apolipoprotein A-1 and Apolipoprotein B-100 by using* Phage-displayed Random Peptide library, Chi-Hoon Kim, Hanyang Univ., 1997).

The mimetic peptide for the epitope of apolipoprotein B-100 as disclosed herein, can be selected from peptides of SEQ.ID. No.1, SEQ.ID. No.2, SEQ.ID. No.3 or the mixtures thereof.

The mimetic peptides of the present invention may be used in the form of concatemer in order to improve their antigenic determinant. As an embodiment of the present invention, two or more mimetic peptides may be linked with each other. The concatemer composed of three(3) to fifteen(15) peptides is desirable. More preferably, the concatemer of the present invention comprises four(4) peptides of SEQ.ID. No. 1.

"Concatemer" of the above mimetic peptide of the present invention, refers to a polymer wherein the ends of the above mimetic peptides are linked with each other.

"The modified peptide" of the above mimetic peptide of the present invention, refers to mimetic peptides variants which can be recognized by monoclonal or polyclonal antibody for apolipoprotein B-100. Such variants include substitutions, deletions, addition, and chemical substitutions of one or more amino acid from the mimetic peptide of the present invention.

Yet another object of the present invention is to provide a method for preparing mimetic peptide concatemer and modified peptides thereof, which comprises: i) a step for inserting DNAs which encodes the above mimetic peptide concatemer and the modified peptide thereof into a vector, ii) a step for transforming the above vector to host cells, and then incubating them, and iii) a step for isolating the above mimetic peptide concatemer or modified peptides thereof from the above host cells.

Formulation of the vaccine composition may be prepared through any conventional method with the mimetic peptide concatemer or modified peptides thereof of the present invention. In the process for preparing the above formulation, preferably, the composition wherein the active compound mixed or diluted with immune adjuvant, drug for reinforcing immunity, carrier, expient and diluent, is selected from the group consisting of tablet, pill, granule, powder, cachet, suspension, emulsion, liquid, syrup, aerosol, soft or hard gelatin capsule, sterilized liquid for injection, sterilized powder and the likes.

Immune adjuvant which may be employed in the composition of the present invention, is a sort of proteins containing the epitope of T cell (e.g. surface protein of hepatitis B virus), inert carrier such as aluminium salt, bentonite, latex, acrylic particle and the like; hydrophobic antigen (e.g. lipid), water-oil and oil-water emulsions, depot former (e.g. polysaccharide), T cell activator such as PPD, polyadenine, polyuracil and the likes; B cell activator (e.g. B cell mitogen), sulfactant such as saponin, lysolecithin, retinal, quil A, liposome and the likes; material for reinforcing activity of macrophage; and alternative pathway complement activators such as inulin, zymosan, endotozin, lebamisole, C. parvum and the likes.

"Carrier protein" of the present invention means a pharmaceutically allowable material such as protein or aluminium salt which can transport the mimetic peptide, concatemer and modified peptides thereof of the present invention through blood stream.

Aluminium salt, phenoxyethyl ethanol, water, physiological salt solution, lactose, dextrose, sucrose, sorbitol, manitol, calcium silicate, cellulose, methyl cellulose, amorphous cellulose, polyvinylpyrolidon, metylhydroxy bezoate, propylhydroxybezoate, talc, magnesium stealate and a mineral oil may be used as suitable carriers, expients or diluents in the composition of the present invention.

In addition, the composition of the present invention may further comprise a filler, an anti-cohesive agent, a lubricant, a moisturizer, a perfume, an emulsifier and an antiseptic.

The composition of the present invention may be formulated by the conventional method well-known in this field to induce immune response on mammal through one (1) time or more inoculation.

The vaccine composition for the treatment of obesity of the present invention may be administered through various routes such as oral, dermal, intradermal, venous or muscular administration, preferably, the intradermal administration.

The effective dose of the vaccine composition of the present invention, is 0.1 to 10 µg (active peptide)per kg of body weight, preferably, 0.5 to 1.0 µg per kg. However, the actual dosage of the active principle of the vaccine composition may be determined depends on several factors such as condition of immunity, administration routes, condition of patient, age, sex, body weight and the like. Therefore, the ranges of the said dosage amount do not limit the scope of the present invention in any way.

The primary pharmaceutical effect of the vaccine composition of the present invention, is to prevent or treat obesity through the mechanism that human antibody induced by mimetic peptide, concatemer or modified peptides thereof, binds on the epitope of apolipoprotein B-100 on the surface of LDL, and thereby hinders sterically and inhibits lipase from the generation of fatty acids, the major etiological factor for obesity.

In addition, the vaccine composition of the present invention has an effect also on suppressing hyperlipoidemia through the mechanism wherein LDL is detected and removed easily by macrophage through the opsonization caused by the human antibody induced by mimetic peptide, concatemer or modified peptides thereof and conjugated on the epitope of apolipoprotein B-100 on the surface of LDL.

Another pharmaceutical effect of the composition of the present invention, is to prevent or treat obesity by inhibiting accumulation of lipid as like cholesterol of free fatty acid in cell through the mechanism wherein human antibody induced by mimetic peptide, concatemer or modified peptides of the present invention binds to the epitope of apolipoprotein B-100 on the surface of LDL, and thereby prohibits LDL from binding specifically to LDL receptor exposed on cell surface.

### Brief Description of the Drawings

The above objects and other advantages of the present invention will become more apparent by describing in detail a preferred embodiment thereof with reference to the attached drawings, in which:
Fig.1a to 1d represent the structures and compositions of vector for expressing the mimetic peptide of the present invention. Fig 1a represents the structure of leader cassette, Fig. 1b represents the structure of LB cassette, Fig lc represents the structure of BL cassette and Fig 1d represents the structure of pBX4 expression vector.
Fig.2 represents the procedures for preparation of pBX1 and pBX4 vector for expressing the mimetic peptide of the present invention.
Fig.3 represents the result of polyacrylamide gel electrophoresis(PAGE) for the identification of LB cassette.
Fig.4 represents the result of PAGE for the identification BL cassette incorporated in plasmid pBlue-BL.
Fig.5 represents the result of PAGE for the confirmation of the direction and the number of copy of DNA inserted in plasmid pBX1 and pBX3.
Fig. 6 represents the result of western-blotting for identification of the expressed PB1₄ peptide.
Fig. 7 represents the result of sodiumdodecyl sulfate(SDS) PAGE for confirmation of the purified PBl₄ peptide.
Fig. 8 represents the result of western-blotting for confirmation of the reactivity of the purified PBl₄ peptide against anti-PB1₄ serum.
Fig. 9 represents the result of ELISA for measurement of the avidity of the antibody of mouse induced by PBl₄ peptide.
Fig. 10 is the graph which illustrates the suppressing effect of PB1₄ on the increase of body weight of mouse.
Fig. 11a and 11b are the graph illustrating the change of body weight of mice depends on administration of PB1₄ vaccine of the present invention in 20 weeks after injection of drug which can destroy of hypothalamus.
Fig. 12 is the graph which represents the effect on the concentration of lipid in blood serum according to the injection of PB1₄ vaccine.

### Best mode for carrying out the present invention

Hereinafter, the present invention will be described in more details. However, the present invention explained in below, is given only for the explanation of embodiment of the present invention and not intended to limit the scope of the present invention.

### Example 1: synthesis and annealing of oligonucleotide

The oligonucleotides were chemically synthesized at Genemed Synthesis, Inc. (San Francisco, CA, USA) in accordance with the sequence requested from the present inventor. In order to phosphorylate the 5' end of oligonucleotides, 50µℓ of 100pmol/µℓ oligonucleotide was incubated with 10µℓ of 10mM ATP, 3µℓ of 10U/µℓ T4 polynucleotide kinase (Takara, Otsu, Japan) and 7µℓ of 10X kinase buffer for two (2) hours at 37 °C .

Each of 10µℓ aliquote of above phosphorylated oligonucleotides were mixed together, heated at 80°C for 5 min. and then chilled very slowly to the room temperature thus were annealed to the specific pairing between complementary strands.

### Example 2: ligation

Ligation mixture was prepared by mixing 1µℓ of vector DNA, 5µℓ of insert DNA, 1µℓ of T4 DNA ligase(NEB, Beverly, MA, USA), 1µℓ of 10X enzyme reaction buffer solution(NEB, Beverly, MA) and 2µℓ of distilled water, and then incubated at 16°C during the night and then incubated.

### Example 3: construction of pBX expression vector for expression of mimetic peptide of apolipoprotein B-100

### Step 1: design of the vector

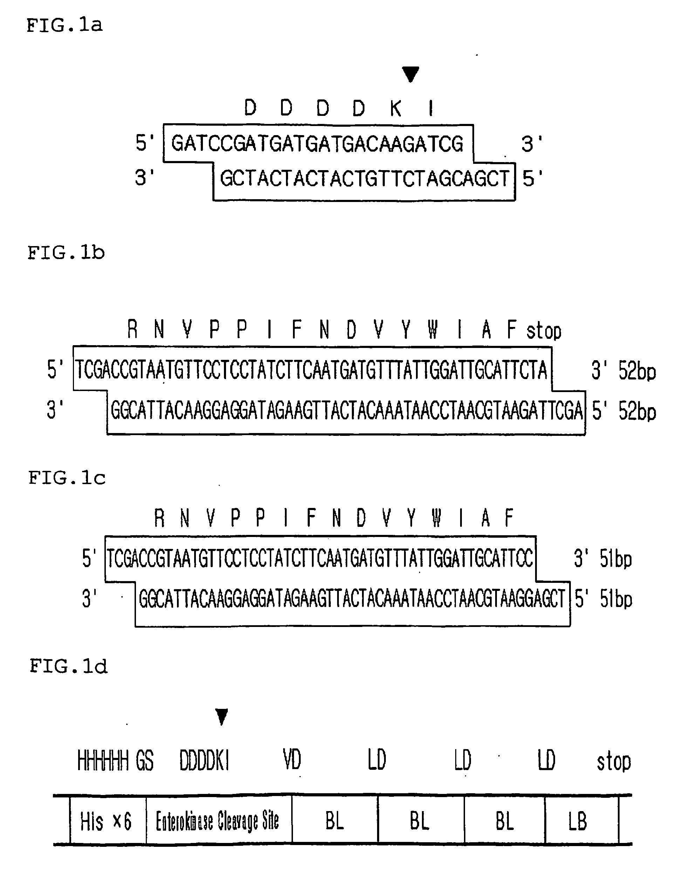

The plasmid vector for expression of mimetic peptide generally comprises a leader cassette and one or more PB1 peptide gene. As depicted in Fig. 1, the plasmid pBX1 which comprises one (1) PB1 gene was prepared by cloning the leader cassette (Fig. 1a) at the multicloning site of pQE30 plasmid(Qiagen, Hilden, Germany). The resulting plasmid was digested with *HindIII* and *SalI* and the small fragment was replaced by the LB cassette (Fig .1b) to give the plasmid pBX1 which is good for the insertion of multiple numbers of BL cassettes (Fig.1c) easily.

Meanwhile,the plasmid pBluescript II SK+ was cut with SalI and XhoI and ligated with BL cassettes in which single to multiple BL cassette insertions occurred randomly. The expected repeats of the PB1 peptide genes in the plasmid pBlue-BL were selected and cleaved oit and subcloned into pBX1 (Fig.1d).

### Step 2: Preparation of vector for expression of PB1 mono-peptide

Leader cassette was prepared by annealing SEQ. ID. No.10 and SEQ. ID. No.11 oligonucleotide synthesized through the same process as Example 1 and 2. Then, the pQE30 vector (Qiagen, Hilden, Germany), which had been cleaved by SalI and *BamH*I, was ligated with the above leader cassette in order to prepare pQE-Leader plasmid. As a result of expression of the above pQE30 vector, six histidine residues were additionally incorporated to the N-terminus of the expressed protein in order for the protein to be purified easily. The above leader cassette was designed to comprise a recognition site (DDDDKI; SEQ. ID. No.12) for enterokinase in order to reduce the additional amino acid to a minimum.

According to the process of Example 1, four oligonucleotides of which sequences were represented by SEQ. ID. No. 4 to 7, were synthesized, phosphorylated, and then, annealed with complementary oligonucleotides respectively in order to synthesize the LB cassette of Fig. 1b(SEQ.ID. No.13 and 14). Forty micro-litter of the annealed oligonucleotides were mixed with 3µℓ of 1 U/µℓ T4 DNA ligase, 5µℓ of 10X enzyme buffer and 2µℓ of distilled water to prepare ligation mixture. Then, the ligation mixture was incubated during the night to link the oligonucleotides each other.

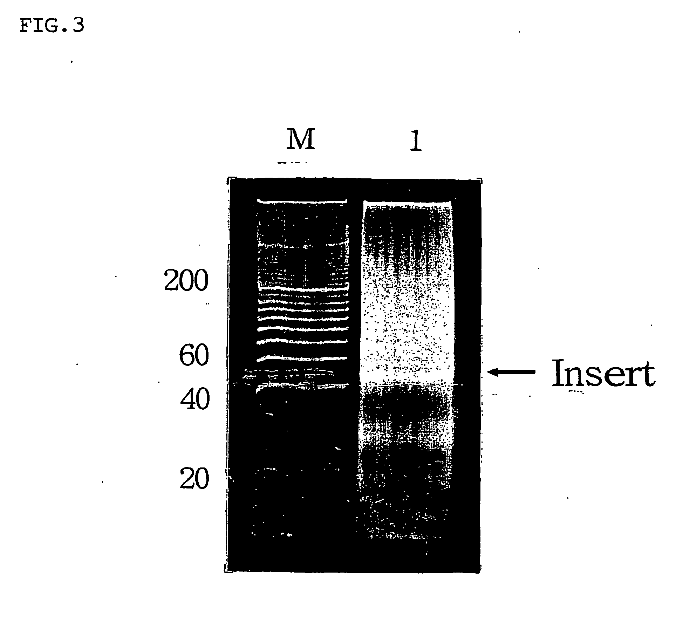

After the reaction being completed, the reaction mixture was loaded onto 20% polyacrylamide gel and electrophoresed. The LB cassette(52 bp oligonucleotides) (Fig. 3) was identified by dying the gel with ethidium bromide (EtBr).

In Fig. 3, lane M was a mark for 20 bp ladder DNA, and lane 1 was the reaction solution. LB cassette was obtained from the gel by means of QIAEX II gel extraction kit(Qiagen, Hilden, Germany).

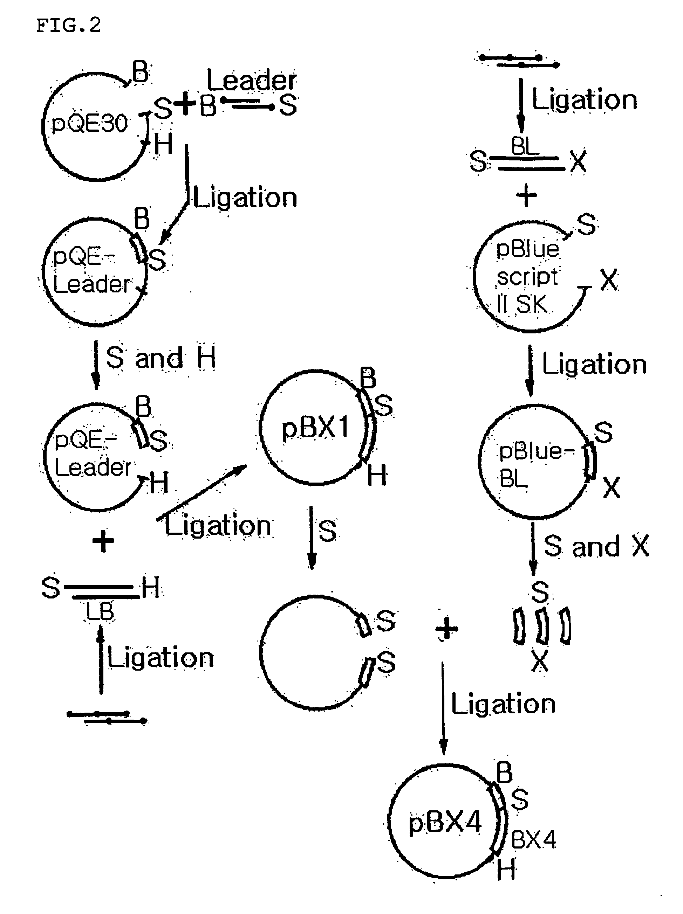

The above pQE-Leader was cleaved by HindIII and SalI, and then ligated with the above LB cassette according to Example 2 in order to prepare expression vector for PB1 mimetic peptide. The prepared expression vector was named pBX1 and the peptide expressed by the vector was named PB1₁ peptide (refers to Fig.2) .

### Step 3: preparation of expression vector for PB1₁ peptide concatemer

According to the process of Example 1, four oligonucleotides of which sequences shown at SEQ. ID. No. 4, 5, 8, and 9, were synthesized, phosphorylated and then, annealed with complementary oligonucleotides respectively in order to synthesize BL cassette of Fig. 1c(SEQ.ID.No.15 and 16). Subsequently such oligonucletides were ligated with each other like the process of Step 2 and then were loaded onto 20% polyacrylamide gel for electrophoresis. Fifty five bp oligonucleotide(leader cassette) was identified by staining the gel with EtBr. BL cassette was obtained from the gel through QIAEX II gel extraction kit and cleaved by SalI and *Xho*I.

Meanwhile, a pBluescript II SK(Stratagene, La Jolla, CA, U.S.A) was cleaved by *Sal*I and *Xho*I. The vector was electrophoresed on 0.8% agarose gel, and obtained by means of QIAEX II gel extraction kit (Qiagen, Hilden, Germany).

Same ligation reaction as Example 2 was carried out to obtain pBlue-BL plasmid by using 5µℓ of BL cassette DNA and 1µℓ of the above cleaved-vector DNA.

The pBlue-BL was cleaved by SalI and XhoI, and the BL cassette was extracted. Plasmid pBX2 was prepared by inserting such BL cassette into the *Sal*I site of the pBX1 vector prepared in Step 2. In addition, pBX3 and pBX4 vector were prepared by changing the number of the BL cassette which inserted into the Sal I site of the pBX1 vector from two (2) to three(3)(refers to Fig. 2).

The peptides expressed from the pBX2, pBX3 and pBX4 vector, were a concatemer which comprises two(2) to four(4) PB1 peptides. They were named PB1₂, PB1₃ and PB1₄ respectively.

### Step 4: Identification of the insert

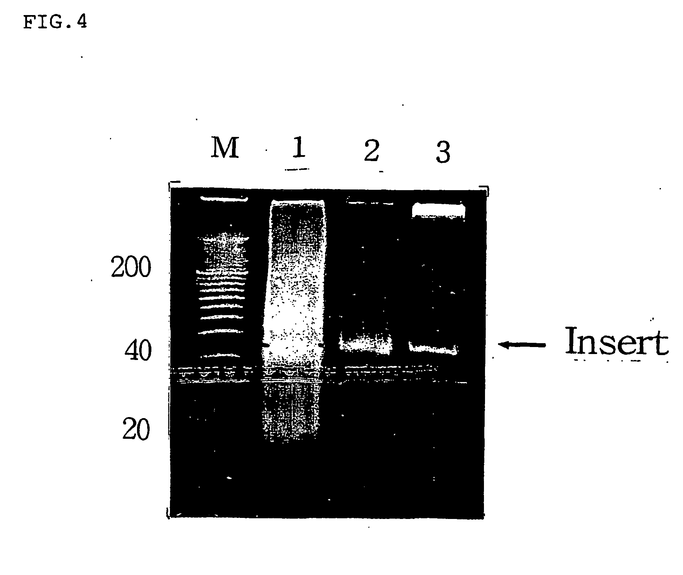

Host cell (*E. coli* M15 [pREP4]; Qiagen, Hilden, Germany) was transformed with the pBlue-BL plasmid and spread onto 1% agar plate, and then incubated for 16 hours at 37°C so that colonies of *E. coli* could be formed. One of the colonies which had been formed on the agar plate, was inoculated in 10mℓ of LB medium and incubated with shaking at 37°C for sixteen(16) hours, and then the plasmid was isolated through DNA purification system(Wizard PLUS SV DNA miniprep DNA purification system; Promega, Madison, W1, U.S.A). The plasmid harvested from the transformed *E. coli* was incubated with SalI and XhoI restriction enzyme in order to be cleaved at 37°C for one (1) hour, and analyzed through 20% PAGE(Fig. 4). In Fig. 4, lane M represents 20 bp ladder DNA, lane 1 represents oligonucleotide product obtained from Step 3, lane 2 represents BL cassette DNA isolated by 20% PAGE from step3 and lane 3 represents recombinant pBlue-BL plasmid treated with restriction enzyme. As shown in Fig. 4, it was confirmed that the pBlue-BL plasmid contained BL cassette.

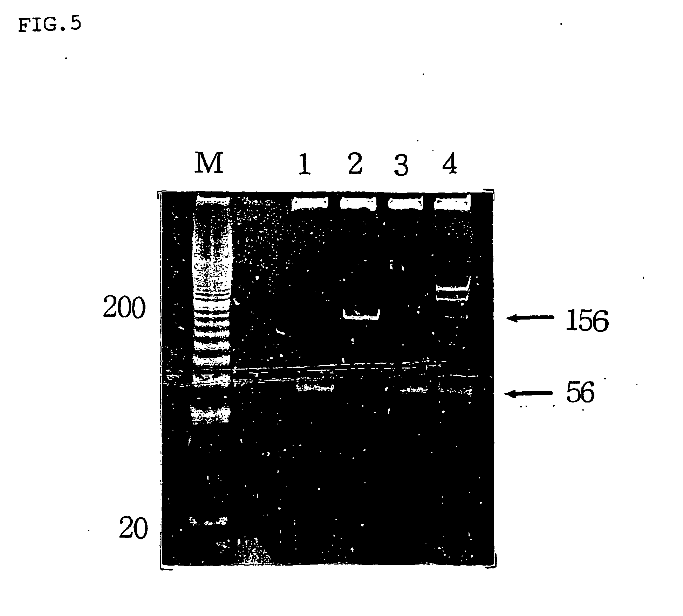

*E. coli* (M15[pREP4]) was transformed with pBX1 or pBX3 plasmid, and the plasmid DNA was isolated as explained above in order to confirm the number and orientation of the DNA cassette inserts. The isolated plasmid was cleaved by *Sal*I and HindIII restriction enzyme, and analyzed through 20% PAGE(Fig. 5). In Fig. 5, lane M represents 20 bp ladder DNA, lane 1 and 3 represent the pBX1 plasmid containing an LB but not BL cassette, lane 2 represents the plasmid harboring one LB and two BL cassettes with the right direction. On the other hand, lane 4 represents the plasmid having one LB and two BL cassettes, however, with reversed orientation. As shown in Fig. 5, how many B cassettes (BL or LB cassette) were inserted and what direction they were inserted into the pBX vector, could identify restriction enzyme mapping.

In addition, the DNA sequence of the B cassettes incorporated into the plasmid which had been harvested from the transformed *E. coli,* was confirmed to be identical to the designed sequences. The plasmids were prepared by Wizard *PLUS* DNA miniprep kit and were sequenced by using Sequennase(Ver. 2.1) DNA sequencing kit(Amersham, Cleveland, UK).

### Example 4: Expression of PB1₄ peptide in E.coli and its purification.

### Step 1: confirmation of expression of PB1₄ peptide

To confirm the expression of PB1₄ peptide, three kinds of transformed *E. coli* M15[pREP4] were cultivated on LB agar broth containing amphicillin and kanamycin. One *E.coli* M15[pREP4] was transformed with the plasmid pBX4, another was mock transformed with pQE30, and the other was not transformed *E.coli* M15[pREP4]. Each colonies formed from the solide culture was inoculated respectively in liquid LB culture medium which contained 100µℓ/mℓ amphicillin and 25µℓ/mℓ kanamycin, and incubated overnight. The culture was incubated at 37°C for one (1) hour with shaking until the O.D value reached in the scope of 0.5 to 0.7 at 600nm. Thereafter, 1 mM isopropyl-thio-β-galactopyranoside (IPTG) was added to the culture medium to facilitate the expression of the recombinant protein, and additional cultivation was made at 37°C for five(5) hours. 1mℓ of the culture medium was taken and centrifuged at 14,000rpm for two (2) min. to precipitate the bacterial cells. The cell pellet was suspended in 50µℓ of 2X SDS solution[100mM Tris-Cl pH 6.8, 20% glycerol(w/v), 4% SDS(w/v), 2% 2-mercaptoethanol, 0.001% bromophenol blue] to apply in SDS-PAGE. The suspended solution was heated at 95 °C for five (5) min., and then 10µℓ of the solution was loaded on the well of the casted gel and electrophoresed at 20mA for five(5) hours(Mighty Small II, Hoefer, USA). Acrylamide concentrations of the stacking gel and resolution gel which were employed, were 5% and 15% respectively, and pre-stained standard SeeBlue(250Kda to 4kDa; NOVEX, San Diego, CA, U.S.A) or wide-ranging standard Mark12 (200kDa to 2.5kDa) were used as a standard size marker protein. After electrophoresis, the gel was stained with Coomassie brilliant blue R-250 for one (1) hour and dyed destained with decolorizing solution (5% Methanol and 7% acetic acid) for ten(10) hours.

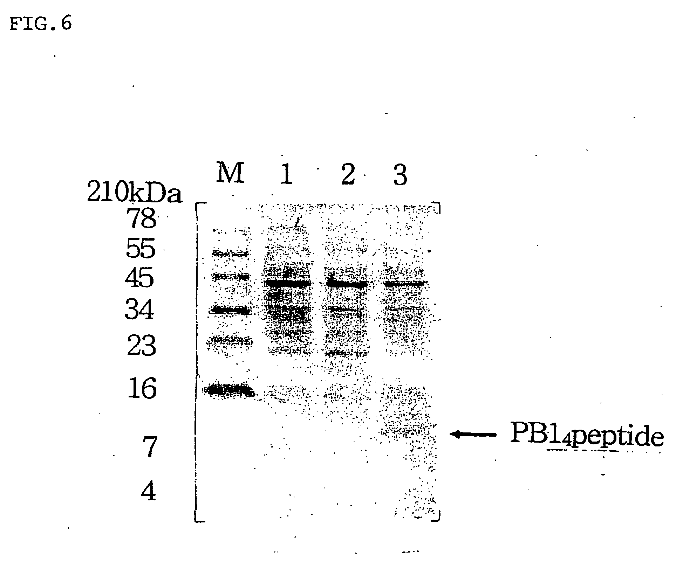

To confirm that the expressed protein is PB1₄ peptide, the proteins in the electrophoresis gel, western-blotting was carried out using anti-PB1 rabbit antibody (Fig. 6). Antiserum was produced by immunizing ovalbumin conjugated which was PB1 peptide chemically synthesized by Bio-Synthesis, Inc.(Lewisville, TX, USA). In Fig. 6, lane M represents the pre-stained standard SeeBlue label, lane 1 represents medium used for incubation of *E.coli* M15 [pREP4] which was not transformed, lane 2 represents medium used for incubation of *E.coli* M15 [pREP4] which was transformed with pQE30 vector, lane 3 represents medium used for incubation of *E.coli* M15 [pREP4] which was transformed with pBX4 vector.

As depicted in Fig. 6, only the pBX4 transformed *E.coli* expressed the recombinant PB1₄ peptide represented a specific immunity with anti-PB1 mouse serum.

### Step 2: Identification of solubility of the expressed peptide

*E.coli* M15 [pREP4] which had been transformed with pBX4 vector, was incubated as the same method of Step 1. 10ml of the culture medium was taken and centrifuged to harvest the cells. The cell pellet was suspended in 5ml of cell lysis solution (300mM NaCl, 50mM NaH₂PO₄, 10mM imidazol pH 8.0) to obtain natural protein from the cell. After being chilled, the pellet-suspended solution was sonicated 20 cycles with ultrasonic wave in order to lyse the cells. The supernatant was taken by centrifuge at 4°C, 10,000rpm for 30 min.. The same volume of 2X SDS solution was mixed with solution and SDS-PAGE was carried out as the same method described in Step 1. After boiling each solution at 95°C for 5 min. As a result of SDS-PAGE, it was confirmed that PB1₄ peptide could be isolated and purified from the soluble extract A in that it was contained in the insoluble crude extract B.

### Step 3: purification of PB1₄ peptide

### Step 3-1: affinity chromatography

Ni-NTA resin for purifying His-tagged proteins, was used to purify the recombinant peptide in Step 1. The affinity chromatography using attractive force between Ni⁺ saturated in resin and histidine residues at the end of the expressed protein, is a well known method for purifying interest protein easily.

First of all, *E.coli* M15[pREP4] which had been transformed with pBX4, was inoculated in 11 of LB culture medium and incubated at 37°C to the extent that O.D value was over 0.6 at 600nm. The ratio of LB culture medium to pBX4 vector was fifty (50) to one (1). IPTG was added in a final concentration of 1mM and incubated again for five (5) hours. After incubation, the cell pellet was obtained by centrifuging the culture medium at 6,000 Xg for 30 min, and the pellet was stored at -70°C over night. The pellet thawed in ice, was suspended in dissolving solution(300mM NaCl, 50mM NaH₂PO₄, 10mM imidazol pH 8.0) wherein 5mℓ of dissolving solution per 1 g of the pellet, was used. Cells are lyzed by sonication as the method of Step 2, and then centrifuged at room temperature at 10,000 Xg for 30 min.. The same volume of buffer (8M urea, 0.1M NaH₂PO₄, 0.01M Tris-HCl pH 8.0) as the pellet, was added for re-suspending the cellular debris and for denaturing proteins there in, and the pellet-suspended solution was treated with brief ultrasonic wave so that more proteins could be dissolved in buffer. The suspension was centrifuged at 8,000rpm for 30 min. to remove cellular debris which had not been solubilized in 8M urea. To the 4mℓ of the supernatant above, 1mℓ of Ni-NTA resin was added at 4°C and shook 200rpm for 2 hours in order proteins containing His-tag to be captured.

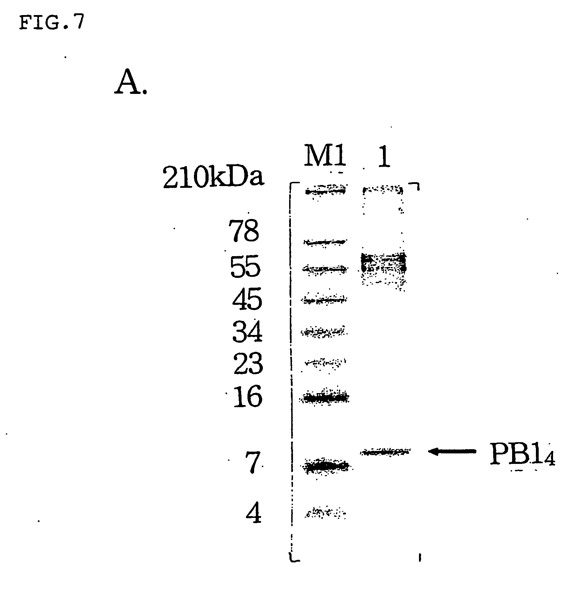

Such supernatant containing protein/Ni-NTA complex was poured carefully into the chromatography column (size 2cm(id)x2.7cm(h)). Excess buffer was drained by opening the cap after the resin had been sunk down. The column was washed with 20mℓ of medium pH buffer (8M urea, 0.1M NaH₂PO₄ 0.01M Tris-HCl pH 8.0) and subsequently 20mℓ of another buffer(8M urea, 0.1M NaH₂PO₄ 0.01M Tris-HCl pH 6.3) in order to wash out proteins which had been non-specifically bound to the Ni-NTA resin. The target proteins containing His-tag were eluted by pouring 5mℓ of low pH buffer(8M urea, 0.1M NaH₂PO₄ 0.01M Tris-HCl pH 5.9) two (2) times and subsequently 5mℓ of strong acid buffer(8M urea, 0.1M NaH₂PO₄ 0.01M Tris-HCl pH 4.5) four (4) times, and then SDS-PAGE was used to confirm the eluted target proteins by using 15% acryamide gel(Fig. 7). In Fig. 7, lane M represents pre-stained SeeBlue size marker and lane 1 represents the purified PB1₄ peptide.

The above purified proteins are dialyzed against PBS(8g/L NaCl, 0.2g/L KCl, 1.44g/L Na₂HPO₄ and 0.24g/L KH₂ PO₄ ) in order to regain their original conformations. Dialysis tube employed, was 3,500 Da in molecular weight cut-off size. During dialysis, 3L of PBS containing 2M urea was used first for 5 hours, and then 5L of PBS without urea was used two (2) times overnight.

### Step 3-2: Hydrophobic chromatography

Hydrophobic chromatography was carried out in order to improve the purity of the PB1₄ peptide which had been obtained in Step 3-1.

Ammonium sulfate was added up to final 20% concentration little by little to the solution containing PB1₄ peptide, which was eluted from Ni-NTA resin in Step 3-1, and then adjusted to pH 7.0. The solution was left for three or more hours after 10% of ammonium sulfate had been melt completely, and then the solution was loaded on the phenyl sepharose column [a filling: Phenyl sepharose Fast Flow resin(Phamacia, Suweden); column size: 1 cm(id) x 3cm(h)].

Each fraction which was eluted from the column by pouring eluting solution(8M urea, 0.1M NaH₂PO₄, 0.01M Tris-HCl pH 6.3) into the column at the flow rate of 0.5ml/min under the reverse gradient of ammonium sulfate from 10% to 0%, was loaded on the gel for SDS-PAGE. The fraction containing PB1₄ peptide was collected and dialyzed in a buffer solution to be desalted, and urea which had been used as a denaturating agent, was removed at the same time.

### Step 3-3: Removal of His-tag

2M urea was added to buffer solution (50mM NaCl, 20mM Tris-HCl, 2mM CaCl₂ pH7.4) which was good for removing denaturating agent and imidazol etc. from the purified his-tagged protein and also for activating enterokinase. The dialyzed PB1₄ peptide which had been obtained from Step 3-2, was dialyzed again by using the above urea containing buffer to desalt the PB1₄ peptide, and during which, the concentration of urea was lowered little by little by repeated dialysis against urea depleted buffer. 3U/ml of enterokinase was added to the PB1₄ peptide-containing solution of which buffer was changed with the said second buffer, and incubated at 23°C. The solution which was taken at every hour, then was analyzed by SDS-PAGE in order to check the amount of his-tag removal from the his-tagged PB1(PB1₄ ^{+his} ) peptide.

### Step 3-4: Ion exchange chromatography

Unwanted proteins and peptides which had been produced as a result of treatment of enterokinase, were removed through ion exchange chromatography.

The solution containing PB1₄ ^{-his} peptide which had been obtained in Step 3-3, was dialyzed in dialysis buffer(2M urea, 0.1M NaH₂ PO₄ , 0.01M Tris-HCl, pH 7.0), and the buffer was exchanged sufficiently. The solution which had been dialyzed, was loaded on the DEAE sepharose resin(Phamacia, uppsala, Sweden). Thereafter, the column was equilibrated with equilibrating buffer (50mM sodium phosphate buffer, 2M urea, pH 7.0) and the peptide was eluted under NaCl concentration gradient from 0 to 1M by using another buffer(50mM sodium phosphate buffer, 2M urea, 1M NaCl) (flow rate:0.5ml/min). Each fraction was recovered and target protein containing fraction were pooled. The presence of PB1₄ ^{-his} peptide was confirmed through SDS-PAGE after concentrating the compartments.

### Step 4: Quantitative analysis of PB1₄

The purified PB1₄ peptide which had been obtained through the same method of Step 3, was quantitatively analyzed through colorimetric analysis by using micro BCA reagent(Pierce, Rockford, USA).

### Step 5: Confirmation of characteristics of the recombinant PB1₄ peptide

Purity of the PB1₄ peptides which had been purified in Step 3 and immunogenicity of them against anti-serum which had been obtained by using synthetic PB1₄ peptide as a antigen, were confirmed through western-blot assay by ECL(Amersham, Cleveland, UK). After SDS-PAGE (Example 2, Step 1), the gel was incubated together with PVDF membrane in buffer(0.3% Tris, 1.5% glycine, 20% methanol) at a constant voltage of 60V for three (3) hours in order for the protein in the gel to be transferred into the PVDF membrane. Then, the blotted membrane was incubated with a 5ml of blocking solution (TBS pH 7.5, 5% skim milk powder(w/v), 0.02% Tween 20) for 1.5 hours, and then was washed three times with TTBS(Tris-buffered saline solution containing 0.1% Tween 20) for 15 min., 5 min. and 5 min. respectively. The antiserum against the peptide PB1(refers to Step 1 of Example 2) was diluted with the TTBS solution in ratio of one (1) to five thousand (5,000), and then incubated with the membrane for 1.5 hours. To confirm the purity of the PB1₄ peptide, anti-serum against PB1₄ peptide (Example 3). After washing the gel with TTBS three times for 15 min., 5 min. and 5 min in turn, the membrane was incubated for 1.5 hours at room temperature with the solution in which alkaline phosphatase-F(ab)'₂-goat anti-mouse IgG (H+L)(Zymed, San Fransisco, CA) was diluted with the TTBS solution in ratio of one (1) to one thousand (1000). The membrane was washed again with TTBS three times, and then colorized by adding BCIP/NBT (5-bromo-4-chloro-3-indolyl phosphate/nitro blue tetrazolium(Sigma)). BCIP/NBT solution was removed using TTBS solution after staining. As a result of western-blotting analysis, the expressed PB1₄ peptide could be recognized with the anti-PB1₄ serum.

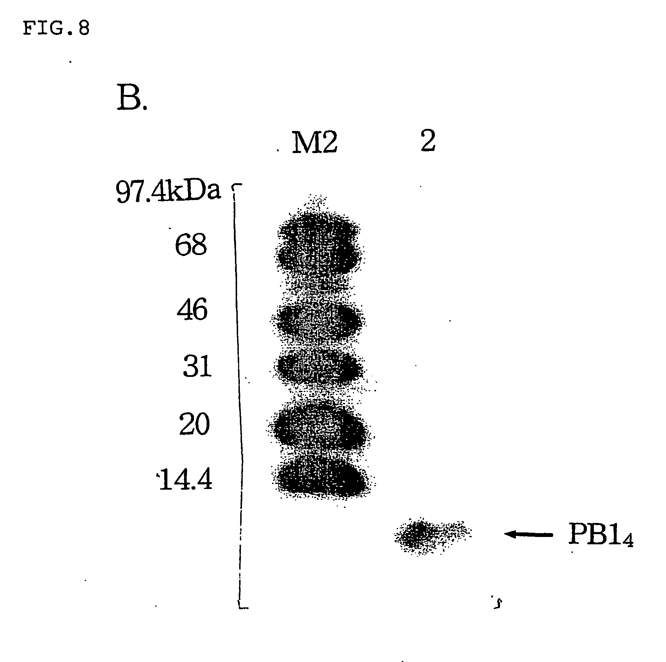

In case of ECL, PVDF membrane(Gelman Science, BioTrace^{R}) was used instead of nitrocellulose membrane. In addition, the first antibody was used in ratio of one (1) to ten thousand (10,000) and HRP-conjugated rabbit anti-mouse IgG (Pierce, Rockford, IL, U.S.A.) was used as the second antibody in ratio of one(1) to ten thousand(10,000). 1 ml of solution A of ECL+Plus western-blot agent (Amersham) per 25 ml of solution B, was used in color reaction. When color was generated sufficiently, the membrane was inserted into film cassette for 5, 10, 20 and 30 seconds respectively to be exposed to the film so that the bands on the gel could be detected (Fig. 8). In Fig. 8, lane M represents a ECL detecting label (Gibco BRL) and lane 1 represents the PB1₄ peptide. As the result from Fig. 8, the expressed PB1₄ peptide could be recognized with the anti-PB1₄ serum.

In addition, the result of western-blot analysis on which the PB1₄ peptide using polyclonal antibody isolated from rabbit serum by using Protein G column (Bio-Rad, USA), gave the same result.

### Example 5: Preparation of anti-PB1₄ peptide mouse antibody

The PB1₄ peptide used herein was the PB1₄ -his peptide from which his-tag was removed, in step 3-3 of example 2.

### Step 1: Libation between PB1₄ peptide and OVA

As a carrier protein, ovalbumin(OVA), was added to the purified PB1₄ peptide in Step 3 in Example 2, in molar ration of one (1) to ten (10), and was incubated for one (1) hour at 4°C. To the PB1₄ peptide-ovalbumin solution, 2% (v/v) glutaraldehyde was added with the same volume, and incubated for one (1) hour with continuous shaking. Then glycine was added to the reaction mixture until final concentration is to be 0.2M to stop the reaction proceeding therein.

After the reaction, the remaining glutaraldehyde-glycine in reaction mixture, were removed by dialysis using MWCO 12,000-14,000 dialysis membrane (Spectrum^{R}, Dominguez, CA, USA).

### Step 2. Immunization of mouse

The peptide with which OVA was linked in Step 1, was concentrated and used to immunize mouse. The amount of the antigen to be injected to the mouse, was 5ug which was the amount of PB1₄ peptide before linked with OVA. The antigen which was emulsified with the same amount of an adjuvant, was injected to intraperitoneum cavity of the mouse in amount of 0.2ml.

Complete Freund's Adjuvant(CFA) was used as the adjuvant of the first injection, and Incomplete Freund's Adjuvant(IFA) was used as the adjuvant at the boosting immunization for two (2) times by two (2) weeks interval. In control mouse, BSA (bovine serum albumin) was injected.

After five (5) days from the final injection, 1ml of blood was taken from mouse by cardiac puncture and the blood was clotted for 30 min. at 37°C. Then, the blood was centrifuged for 30min. at 4°C, 2500xg and the clot was removed from the blood. The supernatant (i.e., blood serum) was incubated overnight at 4°C for the remaining blood coagulants to be concentrated completely, and centrifuged for 20min. at 10,000xg. The resulting supernatant was aliquoted into several tubes. The blood serum which was to be used in experiment, was stored at 4°C, and the remainders were stored at -20°C.

### Step 3. Measurement of avidity of anti-PB1₄ antibody by indirect ELISA

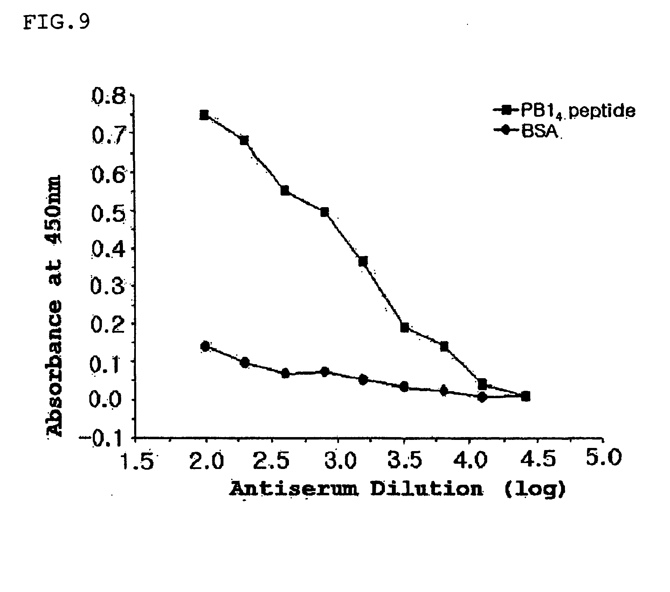

The avidity of the antibody was measured by using blood serum obtained in Step 2. 100ul of PB1₄ peptide was distributed into each well of 96 well micro-titer plate(Flacon: pro-binding) and left alone at 4°C for 6 hours or more, and then washed three(3) times with TTBS(Tirs buffer saline solution containing 0.05% Tween 20). 200ul of blocking solution (1% BSA in TTBS) was added to each well and incubated at 37°C for one (1) hour, and then washed three(3) times with TTBS. 100ul of the isolated serum which had been diluted with the blocking solution in ratio of one (1) from 10² to 10⁵, was added to the reaction solution and incubated at 37°C for 1 hour, and then washed three (3) times with 200ul of TTBS. 100ul of HRP-linked goat anti-rabbit IgG antibody (Pierce, Rockford, IL) which had been diluted with the blocking solution in ratio of one(1) to 10³, was added to the reaction solution and incubated at 37°C for 1 hour, and washed three (3) times with 200ul of TTBS. Solution A of the HRP substrate kit (Bio-Rad) was mixed with solution B of the same in ratio of nine (9) to one (1). 100ul of the resultant mixture was added to the reaction solution and colorized for thirty(30) min., and then optical absorbency for the reaction mixture was measured at 405nm by using ELISA leader (EL312e, Bio-Tek Ins.) (Fig.9). In Fig. 9, it was confirmed that the mouse antibody specific for PB1₄ peptide could be applied to western-blot and ELISA analysis by one (1) thousand fold (3.0 of the X axis in figure).

### Example 6: Anti-obesity effect of PB1₄ vaccine by using a mouse model.

### Step 1:Induction of obesity in a mouse

5 week-old ICR mice (Korea Center for Animal Experiment Ltd., Seoul, Korea) were used herein. The mice were raised in a breeding farm in which temperature was kept from 17 °C to 25°C, and fed to a mixed feed(Sam Yang Feed Ltd., Seoul, Korea, [ingredient: water 11.8% or more, protein 20.0% or more, crude lipid 3.0% or more, crude fiber 10.0% or less, crude ash 10.0% or less, calcium 0.6% or less and phosphorus 0.4% or more]). Goldthioglucose(GTG) was administered to the mice to induce obesity. GTG have a role of inducing desensitization of ventero-medial hypothalamic nuclei (VMH). Therefore, the mice administered with GTG did not feel satiated and always had a desire to eat. The GTG used herein is very unstable compound which is easily degraded in water or moisture. Therefore, 100mg of GTG (Sigma, Inc.) was diluted with 1ml of sesame oil(Sigma Inc.), and was used as the same method of Brecher et al(Brechere G. and Waxler, S. H. Proc. Soc. Exp. Biol. Med., 70: 498-501(1949)) in order for a proper amount of GTG to be administered.

The mice were distributed to prepare a test (twenty (20) mice) and a control group (four (4) mice), and 25ml of GTG was injected to the test group whereas the control was injected with nothing.

Body weight of the mice of the test group was measured prior to experiment and the mice of which deviation of body weight was not significant, were selected and applied to experiment. Body weight of the mice measured after one (1) week after GTG injection, was in the range from 26.5 to 29.5grams.

Seven mice of the GTG-injected group were induced to be obese whereas the remainder was not. The mice which were not induced to be obese, was injected again with GTG, then all the mice were induced to be obese.

All of the obesity-induced mice were distributed in three (3) group. A week later from second GTG injection the PB1₄ peptide was injected to the mice of test group 1 consisting of seven (7) mice as the same method in Step 2 of Example 3. In addition, the mice (test group 2 containing seven (7) mice) of another group of the three, were injected with ovalbumin instead of PB1₄ peptide as a mock experiment, and vaccine was not injected to the other group (test group 3 containing six(6) mice) to induce obesity. On the other hand, 0.2ml of PBS was injected to the control group to be compared with the test groups to confirm the effect of the vaccine of the present invention.

In addition, feed used herein was mixed with the yolk of an egg and dried at 50°C to induce intake of cholesterol so that the level of cholesterol could increase in mouse serum. Feed was also provided enough for disease related to the level of cholesterol to be caused. Body weight of the mouse was measured everyday.

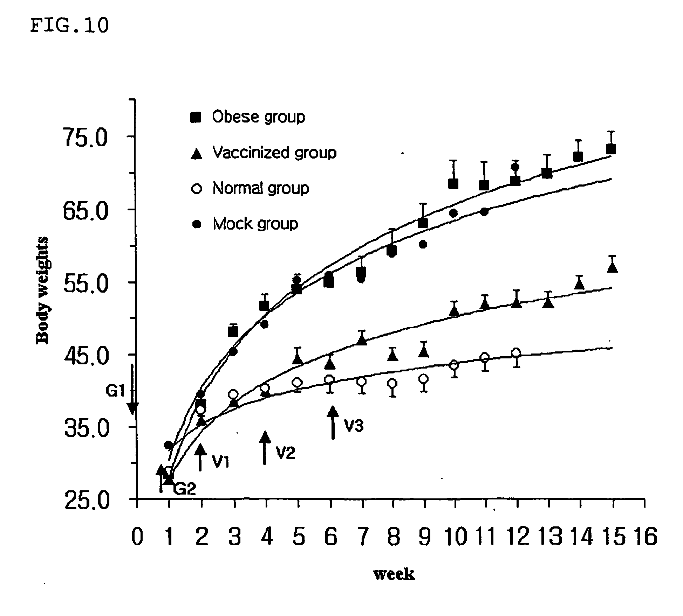

As depicted in Fig. 10, body weight of the vaccine-injected mice of test group 1(-▲-▲-) , increased from 27.7±0.4g to 52.2±1.7g after twelve weeks (12) of GTG injection. The data justify a conclusion that there was no significant difference of an increase in body weight between the test group 1 and the control group (-○-○-) . However, body weight of the mice of both the test group 2(-•-•-) in which ovalbumin was injected after being obese and the test group **(-■-■-)** in which no vaccine was injected after the induction of obesity, increased continuously from 28.3±0.5g to 68.9±2.8g. Therefore, it was confirmed that obesity could be inhibited by injection of PB1₄ peptide vaccine.

In Fig. 10, G1 and G2 represent the time of GTG injection and V1, V2 and V3 represent the time of injection of PB1₄ peptide vaccine.

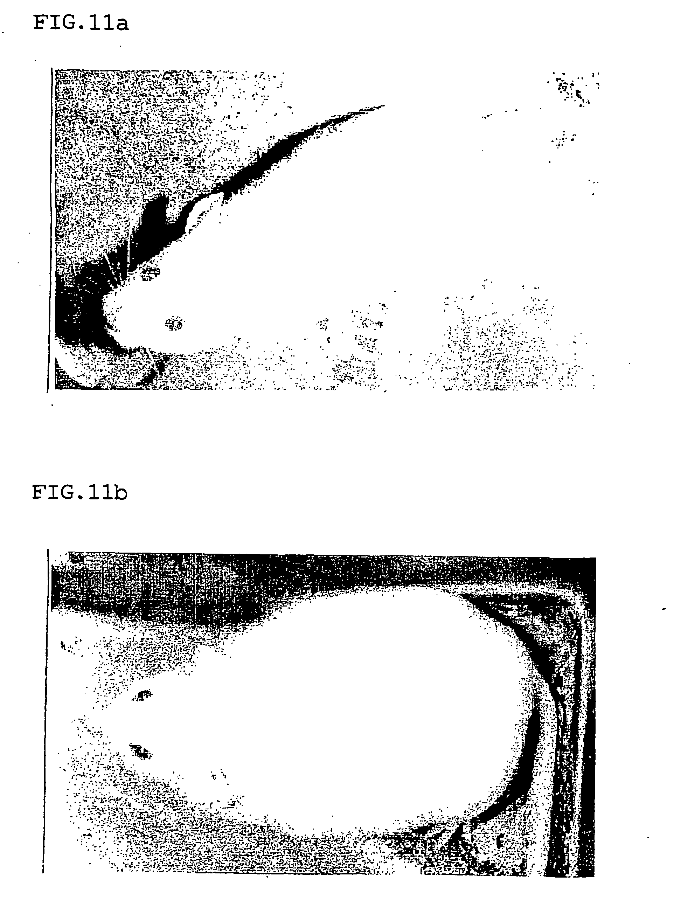

Fig. 11 represents appearance of the obesity-induced mice. 20-week old mouse of the test group 1(Fig.11a: normal mouse) was compared with 20-week old mouse of the test group 3(Fig.11b:obese mouse). As depicted in Fig. 11, it was confirmed that the vaccine of the present invention was effective in inhibiting obesity.

### Step 2: Measurement of the level of cholesterol in blood.

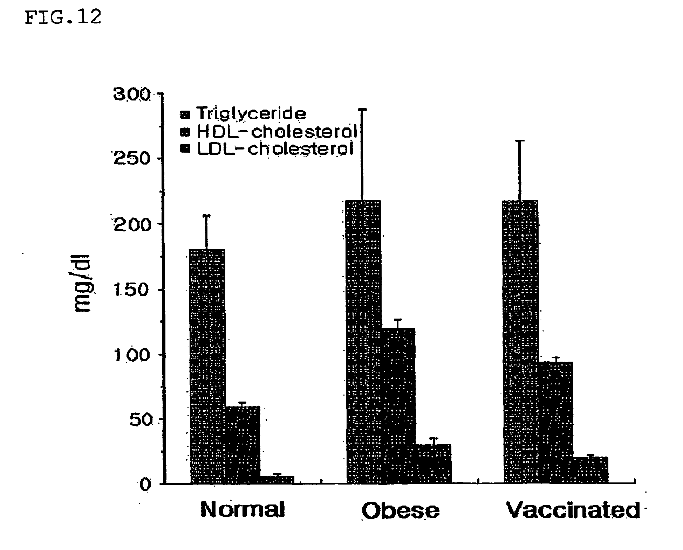

After the first GTG injection, blood cholesterol level of 12-week old mouse of the control group was compared with that of 12-week old GTG-injected mouse of the test group 1 and 2. Concentration of total cholesterol, triglyceride, HDL-cholesterol and LDL-cholesterol was measured through an enzymatic method by using Cholestezyme-V, Triglyzyme-V, HDL-C555 (Shin Yang Chemicals, Seoul, Korea) and LDL-EX kit(Denka Bio-Research, Ltd., Tokyo, Japan). In each experiment, a standard curve for O.D value was prepared by using standard Calibrater-D (Denka Bio-Research, Ltd., Tokyo, Japan) to decrease an experimental error. O.D value for the interest was calculated based on the calibration curve to confirm the concentration and content of the lipid, the result was depicted in Table 1 and Fig. 12.

| | Total cholesterol | TG | HDL-C | |
|---|---|---|---|---|
| Control | 79±3.7 | 180±26 | 59±3.4 | |
| Test group 1 | 118±3.6 | 217±47 | 92±4.7 | 20±1.7 |
| Test group 2 and 3 | 131±8.8 | 218±70 | 119±7.5 | 30±4.5 |
| TG: triglyceride, HDL-C: HDL-cholesterol | | | | |
| LDL-C: LDL-cholesterol | | | | |

As depicted in Table 1 and Fig. 12, as the result of the induction of obesity, it was confirmed that there was no significant difference in the content of cholesterol of both the test group and the control did not increase whereas overall blood concentration of total cholesterol, HDL-C and LDL-C increased in small amount (Fig.12).

### Industrial Applicability

The vaccine composition of the present invention, which contains mimetic peptide for the epitope of apolipoprotein B-100, the concatemers and modified peptide thereof, can be inhibit occurrence of obesity without causing auto-immunity in organism.

Therefore, LDL-related circulatory disease can be treated by vaccine of the present invention more effective than the transitory and high-priced conventional method in which cholesterol metabolism-related enzyme was inhibited.

While the present invention has been particularly shown and described with reference to particular examples thereof, it will be understood by those skilled in the art that various change in form and details may be conceived.

## Claims

1. An isolated concatemer of peptides, wherein said concatemer comprises two (2) to fifteen (15) peptides each having the amino add sequence of SEQ. I.D. NO:1, or the amino acid sequence of SEQ. I.D NO:2 or the amino acid sequence of SEQ. I.D. NO:3.

2. The concatemer or Claim 1, wherein said concatemer comprises four (4) peptides each having the amino acid sequence SEQ. I.D. NO:1 or the amino acid sequence of SEQ. I.D. NO: 2 or the amino acid sequence of SEQ. I.D. NO:3.

3. A vaccine composition for treatment of obesity comprising a peptide selected from the group consisting of a concatemer of peptides wherein said concatemer comprises two (2) to fifteen (15) peptides each having the amino acid sequence of SEQ. ID. No. 1

4. The vaccine composition according to Claim 3 for the manufacture of a medicament for the treatment of obesity wherein the vaccine is to be administred by intradermal injection.

5. The vaccine composition according to Claim 3, wherein said composition is in the form selected from the group consisting of tablets, pills, granules, cachets, elixira, supensions, emulsion, solution, syrups, aerosols, soft or hard gelatin capsules, sterilized injectable solution and sterilized powder.

6. A process for preparing an isolated concatemer of peptides, wherein said concatemer comprises two (2) to fifteen (15) peptides each having the amino acid sequence of SEQ. I.D. NO:1, or the amino acid sequence of SEQ. I.D, NO:2 or the amino acid sequence of SEQ. I.D. NO:3, comprising:
i) inserting, into a vector nucleic acid encoding a concatemer of peptides, wherein said concatemer comprises two (2) to fifteen (15) peptides each having an amino acid sequence of SEQ. I.D. NO:1, or the amino acid sequence of SEQ. I.D. NO:2 or the amino acid sequence of SEQ. I.D. NO:3;
ii) transforming host cells with the vector obtained in step i);
iii) culturing said transformed host cells under conditions that permit expression of the inserted nucleic acid and formation of the encoded concatemer of peptide; and
iv) isolating said concatemer of peptides from the host cell.

## Patentansprüche

1. Isoliertes Concatemer von Peptiden, wobei das Concatemer zwei (2) bis fünfzehn (15) Peptide umfaßt, von denen jedes die Aminosäuresequenz SEQ. I.D. NO:1 oder die Aminosäuresequenz SEQ. I.D. NO:2 oder die Aminosäuresequenz SEQ. I.D. NO:3 aufweist.

2. Concatemer nach Anspruch 1, wobei das Concatemer vier (4) Peptide umfaßt, von denen jedes die Aminosäuresequenz SEQ. I.D. NO:1 oder die Aminosäuresequenz SEQ. I.D. NO:2 oder die Aminosäuresequenz SEQ. I.D. NO:3 aufweist.

3. Impfstoffzusammensetzung zur Behandlung von Obesitas umfassend ein Peptid ausgewählt aus der Gruppe bestehend aus einem Concatemer von Peptiden, wobei das Concatemer zwei (2) bis fünfzehn (15) Peptide umfaßt, von denen jedes die Aminosäuresequenz SEQ. I.D. NO:1 aufweist.

4. Impfstoffzusammensetzung nach Anspruch 3 zur Herstellung eines Medikaments zur Behandlung von Obesitas, wobei der Impfstoff durch intradermale Injektion verabreicht wird.

5. Impfstoffzusammensetzung nach Anspruch 3, wobei die Zusammensetzung ausgewählt wird aus der Gruppe bestehend aus Tabletten, Pillen, Granulat, Cachets, Elixieren, Suspensionen, Emulsionen, Lösungen, Sirups, Aerosole, weiche oder harte Gelatinekapseln, sterilisierte injizierbare Lösungen und sterilisierte Pulver.

6. Verfahren zur Herstellung eines isolierten Concatemer von Peptiden, wobei das Concatemer zwei (2) bis fünfzehn (15) Peptide umfaßt, von denen jedes die Aminosäuresequenz SEQ. I.D. NO:1 oder die Aminosäuresequenz SEQ. I.D. NO:2 oder die Aminosäuresequenz SEQ. I.D. NO:3 aufweist, umfassend:
i) Einbringen von Nukleinsäure in einen Vektor, die ein Concatemer von Peptiden kodiert, wobei das Concatemer zwei (2) bis fünfzehn (15) Peptide umfaßt, die jeweils die Aminosäuresequenz SEQ. I.D. NO:1 oder die Aminosäuresequenz SEQ. I.D. NO:2 oder die Aminosäuresequenz SEQ. I.D. NO:3 aufweisen;
ii) Transformation von Wirtszellen mit dem in Schritt i) erhaltenen Vektor;
iii) Kultivierung der transformierten Wirtszellen unter Bedingungen, die die Expression der eingebrachten Nukleinsäure und die Bildung des kodierenden Concatemers von Peptiden erlauben; und
iv) Isolierung des Concatemers von Peptiden aus den Wirtszellen.

## Revendications

1. Concatémère de peptides isolé, où ledit concatémère comprend deux (2) à quinze (15) peptides ayant chacun la séquence d'acides aminés de SEQ ID NO:1 ou la séquence d'acides aminés de SEQ ID NO:2 ou la séquence d'acides aminés de SEQ ID NO:3.

2. Concatémère selon la revendication 1, où ledit concatémère comprend quatre (4) peptides ayant chacun la séquence d'acides aminés de SEQ ID NO:1 ou la séquence d'acides aminés de SEQ ID NO:2 ou la séquence d'acides aminés de SEQ NO:3.

3. Composition vaccinale destinée au traitement de l'obésité comprenant un peptide sélectionné dans le groupe constitué d'un concatémère de peptides où ledit concatémère comprend deux (2) à quinze (15) peptides ayant chacun la séquence d'acides aminés de SEQ ID NO:1.

4. Composition vaccinale selon la revendication 3 destinée à la préparation d'un médicament destiné au traitement de l'obésité où le vaccin doit être administré par injection intradermique.

5. Composition vaccinale selon la revendication 3, où ladite composition se présente sous une forme sélectionnée dans le groupe constitué de comprimés, pilules, granules, cachets, élixirs, suspensions, émulsion, solution, sirops, aérosols, capsules de gélatine molle ou de gélatine dure, solution injectable stérilisée et poudre stérilisée.

6. Procédé de préparation d'un concatémère de peptides isolé, où ledit concatémère comprend deux (2) à quinze (15) peptides ayant chacun la séquence d'acides aminés de SEQ ID NO:1 ou la séquence d'acides aminés de SEQ ID NO:2 ou la séquence d'acides aminés de SEQ ID NO:3, comprenant :
i) l'insertion dans un vecteur d'acide nucléique codant un concatémère de peptides, où ledit concatémère comprend deux (2) à quinze (15) peptides ayant chacun une séquence d'acides aminés de SEQ ID NO:1 ou la séquence d'acides aminés de SEQ ID NO:2 ou la séquence d'acides aminés de SEQ ID NO:3 ;
ii) la transformation de cellules hôtes par le vecteur obtenu à l'étape i) ;
iii) la culture desdites cellules hôtes transformées dans des conditions qui permettent l'expression de l'acide nucléique inséré et la formation du concatémère de peptides codé ; et
iv) l'isolement dudit concatémère de peptides de la cellule hôte.
